Europäisches Patentamt

(19) European Patent Office

Office européen des brevets

(11) Publication number: **0 006 582**
**B1**

(12) **EUROPEAN PATENT SPECIFICATION**

(45) Date of publication of patent specification: **07.06.89**

(21) Application number: **79102058.9**

(22) Date of filing: **21.06.79**

(51) Int. Cl.⁴: **G 01 N 33/50,** G 01 N 33/96,
C 07 C 103/50

(54) **Method for stabilizing solutions of gamma-glutamyl-p-nitroanilide.**

(30) Priority: **26.06.78 US 919159**

(43) Date of publication of application:
**09.01.80 Bulletin 80/01**

(45) Publication of the grant of the patent:
**07.06.89 Bulletin 89/23**

(84) Designated Contracting States:
**AT BE CH DE FR GB IT LU NL SE**

(56) References cited:
**FR-A-2 308 639**
**FR-A-2 344 570**
**JP-A-51 118 732**
**US-A-3 776 900**

**Sadamasa Minato, Clin. chim. Acta 78, 503-6 (1977)**
**A.I. Vogel, A text-book of practical organic chemistry, 3d ed. (1956), pp. 139-143 and 965**

**CLINICAL CHEMISTRY; PRINCIPLES AND TECHNICS, second ed., 1974, publ. by Harper and Row, New York, USA, R.J.HENRY et al.: "Extraction of cholesterol"**

(73) Proprietor: **Modrovich, Ivan Endre**
**628 Calle Plano**
**Camarillo California 93010 (US)**

(72) Inventor: **Modrovich, Ivan Endre**
**628 Calle Plano**
**Camarillo California 93010 (US)**

(74) Representative: **Patentanwälte Dipl.-Ing. A. Grünecker, Dr.-Ing. H. Kinkeldey, Dr.-Ing. W. Stockmair,**
**Dr. rer. nat. K. Schumann, Dipl.-Ing. P.H. Jakob,**
**Dr. rer. nat. G. Bezold Maximilianstrasse 58**
**D-8000 München 22 (DE)**

EP 0 006 582 B1

Courier Press, Leamington Spa, England.

## Description

This invention relates to a method for stabilizing a solution of γ-glutamyl-p-nitroanilide.

It has been estimated that 25 percent of all in vitro diagnostic tests conducted annually in the Unites States are not reliable. Unreliable tests can result in unnecessary medical treatment, the withholding of necessary treatment and lost income. Because of their high specificity, the use of enzyme determinations has significantly increased during the last few years, and indications are that this trend will continue. However, rigorous quality control measures are required to assure the accuracy and consistency of results. This requirement stems from the fact that the exact nature of enzymes, as well as the mechanisms of their action, remain unknown for the most part.

At present, the most important reason for the unreliability of in vitro diagnostic tests is the instability of the aqueous solution of the organic reagents used. Current methodologies require the use of numerous labile reagents. Due to this problem, rigorous quality control in the manufacture of in vitro diagnostic kits is required, and this quality control is, of course, costly. Moreover, if control if any step in the process is not maintained within a high degree of control standards, the quality of the final product can be reduced materially.

FR—A—2344570 discloses a method for stabilizing a labile coenzyme which is unstable in aqueous media by dissolving it in a dry water-miscible organic solvent to which a desiccant is added.

Sadamasa Minato, Clin.chim. Acta *78*, 503—6 (1977) and JP—A—51—118732 disclose a process wherein a strong acid addition salt of γ-glutamyl-p-nitroanilide is dissolved in an organic solvent, preferably DMSO.

It is the object of the present invention to provide an improved method for stabilizing a solution of γ-glutamyl-p-nitroanilide so the reagent can be provided in a single container with excellent shelf life, where the container can be repeatedly opened for use without substantial degradation of the organic reagent therein. Said object is achieved by a method for stabilizing a solution of γ-glutamyl-p-nitroanilide which comprises the steps of dissolving γ-glutamyl-p-nitroanilide in dimethylsulfoxide or a mixture of dimethylsulfoxide and acetone; adding at lesat 1% by weight of an inert, high-surface-area, particulate desiccant in the solvent, either before or after dissolving the reagent in the solvent, for entrapping water with the desiccant so that the residual water content in the solution is below 0,5% by weight; adding a solubilizing agent selected from boric acid, imidazole, salicylate, and ascorbic acid; and sealing the solution.

The inert, high surface area, particulate desiccant is added to the solvent for entrapping water so that the residual water content of the solution is below about 0.5% by weight, and preferably below about 0.1% by weight.

The dessicant can be added to the solvent either before or after dissolving the reagent in the solvent. The solution can be packaged with the desiccant therein, or the desiccant can be removed from the solution before packaging. Preferably, the desiccant is left in the solution to avoid hydrolysis of the organic reagent when the package containing the solution is repeatedly opened and closed during use.

The preferred solvent for gamma-glutamyl paranitroanilide (GGP) is a mixture of water-free dimethyl sulfoxide (70% v/v) and water-free acetone (30% v/v). The solubilizing agent such as solid boric acid is added to increase the solubility of the reagent in the solvent mixture, preferably before the reagent is added to the solvent mixture, and finally molecular sieves (Mesh 4A, Linde Division of Union Carbide) are added to the solution to serve as the inert desiccant. The solution can then be used for the diagnostic determination of the physiologically important enzyme gamma-glutamyltranspeptidase in biological body fluids, such as human serum or plasma. The reagent is effectively stabilized at refrigerator temperature (2—8°C) for several years as opposed to only several days of maximum stability of the reagent in aqueous solution under identical storage conditions.

According to the present invention, GGP is effectively stabilized in an organic solvent solution by preventing hydrolysis and decomposition of the reagent. This means of stabilization ensures long-term stability in a liquid media. Moreover, close tolerance control in manufacturing can be achieved. The resultant high quality product eliminates the inconvenience of the rigid package size and the high cost of packaging and freeze drying and reagent waste characteristic of prior art products.

The proper dessicant is one that has at least the following properties:

1. it removes water from the solvent mixture;

2. it is not degradatively reactive with the reagent used;

3. it continues to remove water from the solution as the water is introduced, such as by repeated opening and closure of the container; and

4. it does not interfere with the intended use of the reagent.

The desiccant maintains the desired low water content, below 0.5% by weight, preferably below 0.1% by weight. The desiccant must be an efficient water absorber substantially nonreactive with the organic reagent.

The dessicant is preferably a high area hygroscopic agent such as a natural or synthetic molecular sieve having a particle size from 2—16 mesh present in an amount of at least 1% v/v, typically from 5—20% v/v. The amount of surface acea is important since the material acts to absorb water into its pores.

Molecular sieves are zeolites or similar materials whose atoms are arranged in a crystal lattice in such a way that there are a large number of small cavities interconnected by smaller openings or pores of precisely uniform size. Normally, these cavities contain water molecules but, upon heating under a vacuum, this water is driven off without any change in the remaining crystal lattice. The network of cavities and pores can occupy 50% of the total volume of the crystals. Molecular sieves have a strong tendency to reabsorb water and other small molecular weight liquids.

A few natural zeolites exhibit molecular sieve characteristics to a limited degree. Synthetic zeolites are available in several sized (pore openings 3, 4, 5 and 10 angstrom units in diameter) with high capacity for absorption and regeneration even when used at elevated temperatures.

It has been found in connection with the present invention that after the composition has been stabilized in the presence of the organic solvent as well as the inert particulate desiccant, the desiccant can be removed without otherwise materially affecting the stability of the composition. Generally, it has been found that the composition should be stored for a period of at least about 24 hours at room temperature in the presence of the desiccant. During this time, any traces of water are absorbed by the desiccant and, upon removal of the same, there is essentially no water available in the composition. The container with the composition may also be opened for a short time and even though water in the air may enter the upper end of the container, the amount of water is relatively small so that it does not cause by material decomposition of the labile components in the composition.

Generally, the desiccant should be kept in contact with the stabilized solution for a period which depends upon the amount of water which was initially in the solution at the time of preparation. In many cases, it has been found that the desiccant should remain in contact with the solution for about three to four days. This time can be shortened by heating the composition to a point where no decomposition of the labile components will occur. Thus, it has been found that it is possible to heat the composition to about 60°C without affecting the labile component. The important factor is that the desiccant should remain in the solution until there is no more than about 0.5% by weight of water.

It has been found that stability of the composition is not materially decreased even when the desiccant has been removed after the initial stage of stability has been attained.

An advantage of removal of the desiccant from the stabilized solution is improved precision in dispensing solution because the desiccant would otherwise absorb some of the solvent itself, or at least maintain a portion of the solvent on the surface of the desiccant by surface tension. By removing the desiccant it is possible to dispense precise amounts in those cases where quantization of the solution is a critical or important factor.

The order of addition of solvent, reagent, solubilizing agent is, in general, unimportant, but for practical purposes it is preferred to first add and dissolve the solubilizing agent in the solvent, followed by the addition of the reagent. The reagent is then dissolved by stirring and/or application of heat, if necessary, but usually so that the temperature of the solvent mixture does not exceed 50°C or the boiling point of the solution, whichever is lower. This is followed by filtering the solution to remove any debris or undissolved matter, and storing the filtered solution over the desiccant in a tightly capped watertight container.

At time of use, the solution is mixed with aqueous buffers which contain one or more other reactive ingredients, and the resulting solution is used in the same manner as a dry blended or a reconstituted freeze-dried preparation would be used. This technique eliminates the high cost of freeze drying the labile reagents from an aqueous reaction-mixture and then reconstituting the reagent just prior to use. By so doing, this method allows flexibility in packaging and application, and insures product quality by providing a water-based reaction mixture along with the stabilized labile solution, thereby providing and insuring the quality of all components of the product for the clinical diagnostic determination in question.

After the liquid stabilized solution is prepared, it is then dispensed into glass or high density polyethylene bottles containing molecular sieves, which are sealed in an airtight condition. These bottles are typically stored under refrigeration. The projected shelf life of the stabilized organic reagents is up to four years under these conditions without appreciable degradation.

GGP treated according to this invention has long-term stability without any effect on enzymatic reactivity or photometric absorptivity. The invention provides reagents where quality control is assured throughout manufacturing, packaging, storage, and use. The inconvenience of limited package sizes of prior art reagents is eliminated, as are the high cost of packaging, freeze drying, and reagent waste. The liquid reagent systems provide flexibility in use because separation of ingredients is easily accomplished. The liquid system offers better reagent homogeneity and packaging, as well as flexibility in usage, compared to the freeze-dried or dry media preparations.

Stabilized GGP solutions prepared according to the invention have been compared against fresh aqueous GGP solutions. The studies show that aged stabilized reagents prepared according to the present invention and fresh aqueous reagents have comparable accuracy, sensitivity, and precision.

The following examples illustrate the present invention.

Example 1

10 g of boric acid were added to 100 ml of dry dimethyl sulfoxide in a glass beaker. The beaker was covered immediately with parafilm, and the boric acid was dissolved by stirring at room temperature. Thereafter, 4.3 g of gamma glutamyl-p-nitroanilide were added to the mixture and the GGP was dissolved by additional stirring.

The solution so obtained was filtered through filter paper, and the filtered solution was stored over dry molecular sieves (zeolites, Linde Division, Union Carbide Corporation). Approximately 30 beads of molecular sieves, size Mesh 4A (4—8 mm in diameter) were added for each 10 ml of the solution. The solution was stored in a tightly capped amber glass bottle over the molecular sieves. The solution can be dispensed into small amber glass bottles in any quantity size containing approximately 30 beads of Mesh 4A molecular sieves per 10 ml of solution, where the bottqes are maintained tightly capped. The sample was stored at room temperature for a year without significant degradation.

Example 2
A mixture of 30% (v/v) acetone and 70% (v/v) dimethyl sulfoxide was used instead of dimethyl sulfoxide in Example 1. The sample was stored under refrigeration at 2—82C. Storage stability is believed to be up to four years without significant degradation. The addition of acetone prevents the freezing of the mixture under refrigeration temperatures.

Example 3
30 μl of the solution prepared by the method of Example 1 or Example 2 were added to each ml of 0.4 Molar aqueous tris (hydroxymethyl) aminomethane buffer solution, pH 8.2±0.15, containing 160 mM glycyl glycine. The resulting solution was then used for the determination of gamma-GT activity in biological fluids. The procedure for the determination of gamma-GT activity can be performed as follows: to 2.9 ml of the solution above, 100 μl of human serum sample are added, mixed, and the rate of formation of paranitroanilide is followed at 405 nm at constant temperature, such as 37°C. The rate of formation of the paranitroanilide is directly proportional to the activity (concentration) of the diagnostically important liver enzyme gamma-glutamyltranspeptidase (gamma-GT). The reaction scheme involved is as follows:

$$\text{GGP} + \text{glycylglycine} \xrightarrow[\text{pH 8.2}]{\text{gamma-GT}} \text{pNA}$$

$$+ \text{GLU-GLYGLY};$$

paranitroanilide strongly absorbs at 405 nm, whereas GGP does not.

**Claim**

A method for stabilizing a solution of γ-glutamyl-p-nitroanilide which comprises the steps of dissolving γ-glutamyl-p-nitroanilide in dimethylsulfoxide or a mixture of dimethylsulfoxide and acetone; adding at least 1% by weight of an inert, high-surface-area, particulate desiccant in the solvent, either before or after dissolving the reagent in the solvent, for entrapping water with the desiccant so that the residual water content in the solution is below 0.5% by weight; adding a solubilizing agent selected from boric acid, imidazole, salicylate, and ascorbic acid; and sealing the solution.

**Patentanspruch**

Verfahren zur Stabilisierung einer Lösung von γ-Glutamyl-p-nitroanilid, bei dem γ-Glutamyl-p-nitroanilid in Dimethylsulfoxid oder einer Mischung aus Dimethylsulfoxid und Aceton gelöst wird, wenigstens 1 Gew.-% eines inerten, teilchenförmigen Trocknungsmittels mit hohem Oberflächenbereich in das Lösungsmittel entweder vor oder nach dem Auflösen des Reagens in dem Lösungsmittel gegeben wird, um Wasser mit dem Trockenmittel einzuschließen, so daß der Restwassergehalt in der Lösung unterhalb 0,5 Gew.-% ist, ein löslichmachendes Mittel, gewählt aus Borsäure, Imidazol, Salicylat und Ascorbinsäure, zugegeben wird und die Lösung verschlossen wird.

**Revendication**

Procédé de stabilisation d'une solution de γ-glutamyl-p-nitranilide qui comprend les stades de dissolution du γ-glutamyl-p-nitranilide dans du sulfoxyde de diméthyle ou un mélange de sulfoxyde de diméthyle et d'acétone; d'addition d'au moins 1% en poids d'un desséchant particulaire inerte, de surface spécifique élevée dans le solvent, soit avant soit après dissolution du réactif dans le solvent, pour piéger l'eau avec le desséchant de telle sorte que la teneur en eau résiduelle dans la solution soit inférieure à 0,5% en poids l'addition d'un agent solubilisant choisi parmi l'acide borique, l'imidazole, le salicylate et l'acide ascorbique; et de bouchage hermétique de la solution.